Europäisches Patentamt

European Patent Office

Office européen des brevets

(11) Numéro de publication: **0 403 394**
**A1**

## (12) DEMANDE DE BREVET EUROPEEN

(21) Numéro de dépôt: 90401680.5

(22) Date de dépôt: **15.06.90**

(51) Int. Cl.5: **A61B 5/00**

(30) Priorité: **16.06.89 FR 8908013**

(43) Date de publication de la demande:
**19.12.90 Bulletin 90/51**

(84) Etats contractants désignés:
**AT BE CH DE ES GB IT LI NL SE**

(71) Demandeur: **EUROPHOR, SOCIETE ANONYME**
**6, rue Jean Viollis**
**F-31300 Toulouse(FR)**

(72) Inventeur: **Hernandez, Luis**
**12, rue de Tananarive**
**F-31200 Toulouse(FR)**

(74) Mandataire: **Cournarie, Michèle et al**
**Office Blétry 2, boulevard de Strasbourg**
**F-75010 Paris(FR)**

(54) **Sonde de microdialyse.**

(57) Sonde de microdialyse comportant un manchon externe contenant les tubes d'amenée (3) et d'évacuation (4) des produits de dialyse caractérisée en ce que la membrane de dialyse (7, 10, 12) est fixée directement sur le manchon externe (2) à son bord extrême, par collage de la membrane, de façon à éviter une surépaisseur à la jonction de la membrane et du manchon.

FIG.1

## Sonde de microdialyse

L'invention concerne une sonde de microdialyse destinée essentiellement à l'introduction dans les tissus biologiques et plus particulièrement dans le tissu cérébral.

Le phénomène de dialyse est utilisé dans le domaine médical et biologique soit pour prélever dans un tissu vivant les substances secrétées en vue de les analyser a des fins de diagnostic soit pour amener dans le tissu vivant une drogue ou un médicament. Il est bien évidemment nécessaire que les dimensions de la sonde de dialyse soient réduites pour ne pas endommager les tissus biologiques, et ceci est capital lorsqu'on travaille sur le tissu cérébral. C'est pourquoi il s'agit ici plutôt de microdialyse.

Les sondes de microdialyse existant a l'heure actuelle comportent deux tubes de dialyse concentriques ou parallèles et à leur extrémité distale, en contact avec le tissu biologique, soit un dispositif de protection externe (cf. par exemple GB-A-1 595 079) soit une monture qui entoure la membrane de dialyse (FR-A- 2 537 000).

De tels dispositifs de protection de la membrane, qui par elle-même est très fine et fragile, ont l'inconvénient d'endommager les tissus biologiques car ils ne peuvent pas être obtenus avec de faibles dimensions tant pour des problèmes de réalisation de ces dispositifs de protection que pour des problèmes de fixation de la membrane, une telle fixation devant bien entendu être parfaitement fiable.

L'invention résoud le problème en fournissant une sonde de microdialyse du type comportant un manchon externe contenant les tubes d'amenée et d'évacuation des produits, ne nécessitant ni dispositif de protection externe ni monture entourant la membrane de dialyse tout en permettant d'utiliser une membrane fine.

Une telle sonde est telle que la membrane de dialyse est fixée directement sur le manchon externe à son bord extrême , de façon à éviter toute surépaisseur à la jonction entre la membrane et le manchon. Il n'y a donc pas écrasement des tissus au-delà de ce qui est strictement nécessaire au passage de la partie active de la sonde.

Dans un premier mode de réalisation, le manchon est biseauté intérieurement et la membrane est fixée par collage, par exemple à l'aide d'une résine époxy, la découpe de la membrane et sa mise en place se faisant par une technique de poinçonnage en insérant l'extrémité biseautée et encollée du manchon dans une membrane en feuille.

Dans un deuxième mode de réalisation, un tronçon de membrane tubulaire est collé dans le prolongement de l'extrémité éventuellement en biseau du manchon, puis on ferme l'extrémité libre du tronçon tubulaire, par exemple par thermoscellage ou par bouchage avec une résine époxy ou autre.

Dans un troisième mode de réalisation, un tronçon de membrane tubulaire est enfilé autour du manchon biseauté extérieurement, avec ou sans collage selon la hauteur d'enfilage, l'extrémité libre étant fermée comme ci-dessus par thermoscellage ou par un bouchon.

De tels modes de réalisation présentent l'avantage d'être de réalisation facile tout en permettant de fabriquer des sondes de très faible diamètre. Il est en effet possible de réaliser des manchons de diamètre compris entre 100, ou même moins, et 150 μm, alors que les techniques antérieures ne permettent pas de descendre en dessous de 200 μm. En outre dans le cas où la membrane obture de façon plane l'extrémité du manchon (premier mode de réalisation), il est possible d'effectuer la microdialyse au contact de cellules, sans les détériorer, et ceci trouve une application particulière au niveau des amas cellulaires très localisés ou nuclei du cerveau. Le fait d'utiliser une sonde de très faible dimension permet bien entendu d'atteindre des zones inaccessibles, sous peine de détérioration, avec les sondes existantes, ainsi que d'opérer avec une très grande précision tant à l'administration d'un produit qu'à la collecte; en effet dans les sondes classiques, la surface de dialyse est relativement importante et il y a dilution dans ou par le milieu ambiant.

Les membranes utilisées peuvent être des membranes de dialyse opérant la sélection sur la base du poids moléculaire et/ou sur tout autre critère approprié (affinité enzymatique, affinité aux groupements sulfure,) pour ne citer que des exemples bien connus.

D'autres caractéristiques et avantages de l'invention apparaîtront à la lecture de la description détaillée suivante faite en référence aux dessins annexés dans lesquels :

la figure 1 est un schéma en coupe d'une sonde de dialyse incorporant un mode de réalisation selon l'invention, et la figure 1a un agrandissement du montage de la membrane.

La figure 2 est le schéma en coupe d'une membrane de dialyse montée sur une sonde selon un autre mode de réalisation,

la figure 3 est un schéma en coupe du montage d'une membrane de dialyse selon encore un autre mode de réalisation, et

la figure 4 montre le bouchage d'une membrane tubulaire par un bouchon époxy.

Une sonde de dialyse 1 comprend un manchon tubulaire 2, en métal ou en matière plastique, par exemple en acier inoxydable - en particulier lorsqu'il existe un biseau -, dans lequel sont montés, par exemple par l'intermédiaire d'un second manchon 2a de diamètre supérieur, deux tubes d'amenée 3 et d'évacuation 4 (ou vice versa). Ces deux tubes 3 et 4 peuvent être en métal ou dans le cas du tube d'amenée, il peut s'agir d'un capillaire en silice fondue ou en polyimide. Le tube d'amenée 3 se prolonge jusqu'à l'extrémité distale de la sonde tandis que le tube d'évacuation 4 s'ouvre à l'extrémité proximale de la sonde (la disposition inverse étant également possible), l'étanchéité entre les deux manchons 2 et 2a d'une part et les tubes 3, 4 et le manchon 2a d'autre part étant assurée par collage 5, 6 par une résine, par exemple époxy. Bien évidemment les tubes 3 et 4 sont reliés à des dispositifs d'alimentation et/ou d'analyse classiques non représentés, appropriés à l'utilisation que l'on fait de la sonde (dialyse de prélèvement, perfusion de médicaments, etc...)

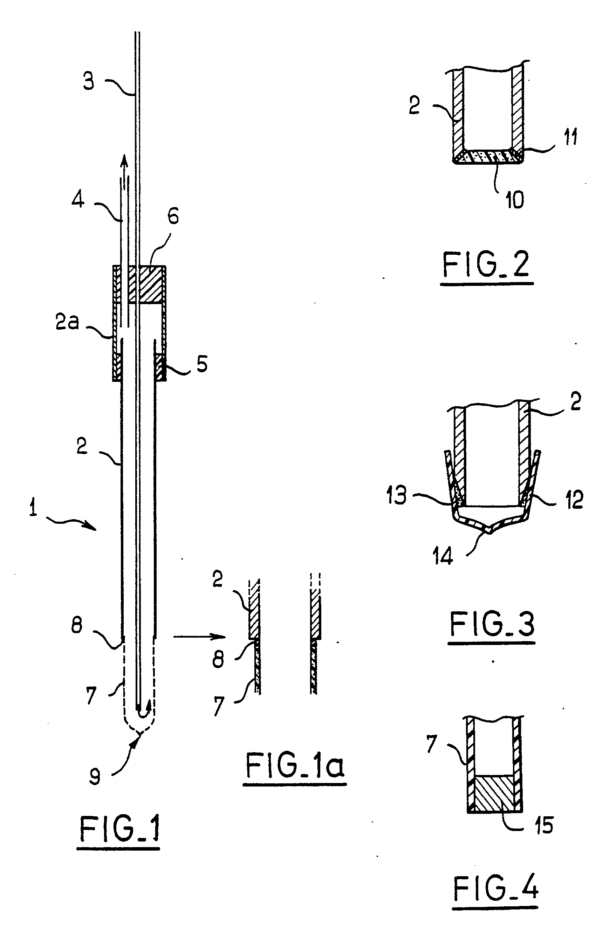

Selon le mode de réalisation des figures 1 et 1a, un tronçon de membrane tubulaire 7 est collé en 8, par exemple à l'aide d'une résine époxy, dans le prolongement du manchon 2. La membrane 7 a sensiblement le même diamètre intérieur que le manchon 2 mais, comme représenté, son diamètre extérieur peut être inférieur. L'extrémité libre du tronçon de membrane 7 est fermée en 9 par thermoscellage.

La fixation de la membrane 7 sur le manchon 2 s'effectue en amenant, sous microscope, en alignement le tronçon et le manchon l'un ou l'autre ou les deux préalablement encollés et en les maintenant en contact le temps que prenne la résine puis on ferme l'extrémité ouverte par pincement à chaud (thermoscellage, 9, 14 figures 1 et 3) ou bouchage par une résine, époxy par exemple (15, figure 4).

L'extrémité du manchon peut être plane ou en biseau intérieur ou extérieur pour donner une plus grande surface de collage avec la membrane.

Lorsqu'on utilise un bouchon, époxy par exemple, le bouchon peut être découpé en biseau, en particulier lorsque la sonde doit être introduite à l'intérieur d'un vaisseau sanguin.

Dans un autre mode de réalisation (figure 2), l'extrémité du manchon 2 est en biseau intérieur et sert de poinçon, après encollage, pour découper dans une membrane en feuille une rondelle 10 qui vient obturer l'extrémité ouverte du manchon et y est maintenue par exemple par une résine époxy 11.

Dans encore une autre réalisation (figure 3), un tronçon de membrane 12 est enfilé à l'extrémité du manchon 2 façonnée en biseau extérieur et est maintenu par collage 13 par une résine, époxy par exemple, au niveau du biseau. Il peut dans certaines applications être avantageux que le manchon 2 en contact avec les tissus biologiques soit recouvert d'une membrane et il suffit alors de donner au tronçon de membrane 12 la longueur nécessaire au-delà du biseau du manchon 2. Si la longueur du manchon est importante, on peut se passer de l'encollage au niveau du biseau. La membrane est représentée légèrement évasée sur la figure 3, pour des raisons de clarté. En fait elle est soit arrêtée au niveau supérieur du biseau soit prolongée au-delà mais en épousant étroitement le manchon de façon à éviter toute surépaisseur. L'extrémité ouverte 14 du tronçon 12 est représentée fermée comme dans le premier mode de réalisation par pincement à chaud mais un bouchon peut aussi être réalisé.

La formation du biseau à l'extrémité d'un manchon métallique s'effectue de la manière utilisée pour affûter les micro-électrodes: l'extrémité est plongée de façon répétée dans un bain d'acide et soumise à un potentiel électrique, avec une protection intérieure ou extérieure selon que l'on désire un biseau extérieur ou intérieur respectivement.

Le manchon de la sonde de microdialyse peut également être réalisé sous forme de cathéter en matière plastique souple, par exemple en silicone élastomère (Silastic®) en raison de sa totale compatibilité biologique; la membrane de dialyse est alors montée à l'extrémité du cathéter en silicone contenant un capillaire en polyimide, le manchon souple pouvant faire office de deuxième tube d'amenée/évacuation. Dans ce cas la membrane de dialyse est sous forme tubulaire, enfilée autour du manchon de silicone et collée par une résine époxy. Un tel dispositif permet d'effectuer une dialyse à distance du point d'entrée dans l'organisme et trouve une application particulière dans la dialyse cardiaque d'animaux de laboratoire qu'il n'est alors pas nécessaire d'immobiliser ou d'anesthésier.

La membrane de dialyse utilisée peut être une membrane classique quelconque, éventuellement modifiée de façon chimique et/ou enzymatique de façon à obtenir les affinités et sélectivités recherchées.

Les sondes de microdialyse selon l'invention peuvent être utilisées pour le prélèvement sélectif de substances excrétées au voisinage de cellules cibles ou pour perfuser à ce voisinage des drogues ou des médicaments ou des inducteurs de réaction ou des indicateurs de réaction (marqueurs, traceurs radioactifs,..). Ceci permet une action plus directe sur la cellule visée qu'une administration par une voie classique.

**Revendications**

1.- Sonde de microdialyse comportant un manchon externe contenant les tubes d'amenée et d'évacuation des produits de dialyse, caractérisée en ce que la membrane de dialyse (7,10,12) est fixée directement sur le manchon externe (2) à son bord extrême, de façon à éviter une surépaisseur à la jonction de la membrane et du manchon.

2.- Sonde de microdialyse selon la revendication 1, caractérisée en ce que le manchon (2) est biseauté intérieurement et la membrane (10) est fixée par collage, la découpe de la membrane et sa mise en place se faisant par poinçonnage d'une membrane en feuille à l'aide du biseau pré-encollé.

3.- Sonde de microdialyse selon la revendication 1, caractérisée en ce qu'un tronçon de membrane (7) est collé dans le prolongement de l'extrémité, éventuellement en biseau, du manchon 2, et l'extrémité libre du tronçon est ensuite fermée (9).

4.- Sonde de microdialyse selon la revendication 1, caractérisée en ce qu'un tronçon de membrane tubulaire (12) est enfilé autour du manchon (2) biseauté extérieurement, l'extrémité libre étant ensuite fermée (14).

5.- Sonde de microdialyse selon la revendication 1, caractérisée en ce que le manchon externe est en matière plastique souple, la membrane de dialyse étant une membrane tubulaire enfilée autour du manchon.

EP 0 403 394 A1

FIG_1

FIG_1a

FIG_2

FIG_3

FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,X | FR-A-2537000 (UNGERSTEDT)<br>* abrégé *<br>* page 6, lignes 10 - 33 *<br>* page 8, ligne 35 - page 9, ligne 5 *<br>* page 10, ligne 32 - page 11, ligne 10 *<br>* page 12, ligne 31 - page 13, ligne 31; figures 4-6 * | 1 | A61B5/00 |
| D,A | --- | 3 | |
| X | US-A-3572315 (CULLEN)<br>* colonne 2, ligne 14 - colonne 3, ligne 25; figures 1, 2 * | 1 | |
| A | --- | 5 | |
| A | PATENT ABSTRACTS OF JAPAN<br>vol. 9, no. 129 (P-361)(1852) 05 juin 1985,<br>& JP-A-60 13242 (NARIYUKI HAYASHI) 23 janvier 85,<br>* le document en entier * | 1 | |
| A | --- <br> FR-A-2248855 (NATIONAL RESEARCH DEVELOPMENT CORPORATION)<br>* page 2, ligne 13 - page 5, ligne 10; figure * | 1, 5 | DOMAINES TECHNIQUES RECHERCHES (Int. Cl.5 )<br><br>A61B |
| A | --- <br> US-A-3893448 (BRANTIGAN)<br>* colonne 2, ligne 47 - colonne 4, ligne 4; figures 1-3 *<br><br>----- | 1, 2 | A61M |

Le présent rapport a été établi pour toutes les revendications

| Lieu de la recherche | Date d'achèvement de la recherche | Examinateur |
|---|---|---|
| LA HAYE | 21 SEPTEMBRE 1990 | CHEN A.H. |